# EUROPEAN PATENT APPLICATION

(11) **EP 2 626 351 A1**
(43) Date of publication of application: **14.08.2013**
(21) Application number: 11830613.3
(22) Date of filing: 03.10.2011
(51) Int. Cl.: C07D 239/47, A61K 31/505, A61P 3/04, A61P 3/10, A61P 13/00, A61P 19/10, A61P 35/04, A61P 43/00

(54) **AGENT FOR INHIBITING EXPRESSION OF LIPID METABOLISM RELATED MRNA**

(30) Priority: 04.10.2010 JP 2010224564; 04.10.2010 JP 2010224563; 04.10.2010 JP 2010224490
(71) Applicant: Kowa Co., Ltd., Nagoya-shi, Aichi 460-8625 (JP)
(72) Inventor: SHIBUYA, Kimiyuki, Higashimurayama-shi Tokyo 189-0022 (JP); OHGIYA, Tadaaki, Higashimurayama-shi Tokyo 189-0022 (JP); MURAKAMI, Takeshi, Higashimurayama-shi Tokyo 189-0022 (JP)
(74) Representative: Blodig, Wolfgang
(86) International application number: PCT/JP2011/072742
(87) International publication number: WO 2012/046681

(57) **Abstract**

The present invention is intended to provide a pharmaceutical product for inhibiting expression of at least one lipid metabolism related mRNA selected from the group consisting of Angptl4 mRNA, SCD-1 mRNA, and SREBP1c mRNA, the present invention is also intended to provide a preventive and/or therapeutic agent for various diseases based on inhibition of expression of at least one lipid metabolism related mRNA selected from the group consisting of Angptl4 mRNA, SCD-1 mRNA, and SREBP1c mRNA, and the present invention relates to an agent for inhibiting expression of at least one lipid metabolism related mRNA selected from the group consisting of Angptl4 mRNA, SCD-1 mRNA, and SREBP1c mRNA, and relates also to a preventive and/or therapeutic agent for various diseases based on the inhibition of the expression of at least one lipid metabolism related mRNA selected from the group consisting of Angptl4 mRNA, SCD-1 mRNA, and SREBP1c mRNA, the agent comprising a compound represented by Formula (I), its salt, or a solvate of any of them as an active ingredient: 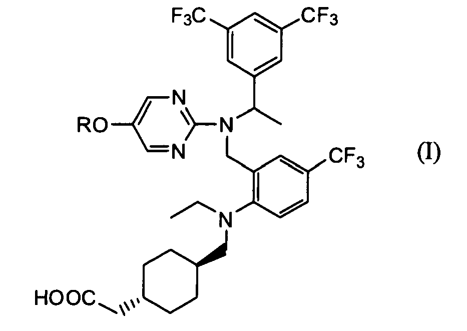 wherein the symbols are the same as those given in the description..

## Description

### Technical Field

The present invention relates to an agent for inhibiting expression of angiopoietin-like protein 4 (Angpt14) mRNA, sterol regulatory element-binding protein 1c mRNA, or stearoyl-coenzyme A desaturase 1 mRNA, which are lipid metabolism related mRNAs. The present invention also relates to an agent for inhibiting the production of angiopoietin-like protein 4, sterol regulatory element-binding protein 1c, or stearoyl-coenzyme A desaturase 1, which are lipid metabolism related proteins.

### Background Art

In recent years, with progress of aging of the Japanese population and westernization of the Japanese diet, diseases such as diabetic, cancer, and osteoporosis are increasing. According to National Health and Nutrition Examination in Japan, 2007, the number of patients who are strongly suspected to have diabetes is estimated at 8,900,000. It is known that development of diabetic symptoms can cause complications such as neuropathy, retinopathy, nephropathy, and dyslipidemia. In addition, with progress of aging society, the number of osteoporosis patients is increasing every year. According to Japan Osteoporosis Foundation, the number of osteoporosis patients in Japan is about 11,000,000. Bone fracture due to osteoporosis has high risk to fall into bedridden life, so that its prevention is important.

Angiopoietin-like protein (Angpt1) is one of secreted glycoprotein having high homology to Angiopoietin which plays an important role in angiogenesis. It is reported that Angptl is different from Angiopoietin and cannot be bind to the Tie1 or Tie2 receptor present in vascular endothelium, and that the biological action of Angptl is different from that of Angiopoietin (see Non-patent Literature 1). Angptl has some family proteins. Among them, Angptl1 to Angpt16 have been identified. Of these identified proteins, Angptl4 is reported to have Lipoprotein Lipase (LPL) activity and action on insulin resistance (see Non-patent Literatures 2 and 3).

Angptl4 inhibits LPL activity, so that inhibition of Angptl4 causes activation of LPL and decrease of the blood triglyceride level (see Non-patent Literature 2). In addition, it is reported that activation of LPL inhibited increase of blood cholesterol levels induced by diabetes in mice with diabetes caused by streptozotocin (see Non-patent Literature 4) . On the basis of these facts, it is expected that inhibition of Angptl4 will decrease blood triglyceride levels, and improve a diabetic complications.

It is also reported that Angptl4 improved bone resorption by osteoclasts (see Non-patent Literature 5). It is also reported that Angptl4 enhanced metastasis of breast cancer to lung (see Non-patent Literature 6). Angptl4 is known to have various biological activities.

In recent years, with the increase of metabolic syndrome, diseases such as dyslipidemia, diabetes, and hypertension are increasing, and patients with fatty liver are also increasing. Some fatty livers are associated with accumulation of fat in the liver despite they are non-alcoholic, and such fatty livers can progress to hepatocirrhosis or liver cancer. This disease is called non-alcoholic steatohepatitis (NASH) and at present has no effective therapeutic agent. Therefore, development of a therapeutic agent is desired (see Non-patent Literature 7).

Sterol regulatory element-binding proteins (SREBPs) are transcription factors which regulate an expression level of proteins related with lipid metabolism. The subtypes of SREBPs include SREBP1a, SREBP1c, and SREBP2. Among them, SREBP1c is abundant in the liver and adipocytes, and controls synthesis of triglyceride in these cells (see Non-patent Literature 8).

Knockout of SREBP1c in ob/ob mice, which are obesity model mice, causes decrease of fatty acid synthase in the liver, which results in decrease of triglyceride levels in the liver. On the basis of these facts, activation of SREBP1c is likely correlated with fatty liver, and there are actual reports that administration of an agent for increasing an expression level of SREBP1c promoted fatty liver (see Non-patent Literatures 8 and 9).

Fatty liver can develop NASH which can lead to hepatocirrhosis or liver cancer, and SREBP1c is suggested to be a risk factor for NASH. It is reported that mice with forced expression of SREBP1c had accumulation of fat in the liver and hepatic fibrosis associated with aging, and showed a NASH-like condition (see Non-patent Literature 10). This model did not progress to liver cancer, but suggests the possibility of progress from fatty liver to NASH caused by activation of SREBP1c. Urgent development of effective agents for NASH is needed, and agents for various targets are under development. SREBP1c likely has potential for becoming a target of drug development.

On the other hand, transcription of SREBP1c is enhanced by hepatitis C virus non-structural protein 2 (HCVNS2), which is considered to contribute to hepatitis C virus-associated adiposis (see Non-patent Literature 11). In addition, it was proved that the increased expression of SREBP1c plays an important role in occurrence of lipid accumulation in the kidney, glomerulosclerosis, tubulointerstitial fibrosis, and proteinuria (see Non-patent Literature 12). Examples of side effects from administration of psychotropic agents include deterioration of myelin sheath function or myelin formation, and malignancy syndrome or extrapyramidal symptoms. It is suggested that these side effects are caused by the increased expression of SREBP1c (see Non-patent Literature 13).

According to the recent reports, obesity causes various diseases such as hyperlipemia, arteriosclerosis, and diabetes. Therefore, anti-obesity agents are under development, and cannabinoid antagonists which act on central nervous system and control appetite, serotonine-noradrenaline reuptake inhibitors, and lipid absorption inhibitors based on lipase inhibition in the small intestine are marketed in several countries. However, these agents are reported to have caused many adverse events and thus have safety problems. Therefore, safe anti-obesity agents are desired, and at present various anti-obesity agents are under development.

SCD-1 (stearoyl-coenzyme A desaturase 1) is an enzyme synthesizing monounsaturated fatty acids from saturated fatty acids, and primarily converts palmitic acid and stearic acid to palmitoleic acid and oleic acid, respectively. It is reported that SCD-1 synthesizes unsaturated fatty acids to protect cells from deficiency of unsaturated fatty acid or toxicity of saturated fatty acids, and play an important role on the skin in development of sebaceous gland (Non-patent Literature 14).

On the other hand, synthesis of fatty acids and triglycerides was inhibited in SCD-1 knockout mice, suggesting that SCD-1 is an important protein for lipid metabolism (Non-patent Literature 15). It is also reported that a ratio of unsaturated fatty acid/saturated fatty acid in human was directly proportional to blood triglyceride levels (Non-patent Literature 16), and knockout of SCD-1 promoted β-oxidation of fatty acids through activation of AMP activated protein kinase (AMPK), whereby energy metabolism in the body was promoted (Non-patent Literature 17). On the basis of these results, anti-obesity action through SCD-1 inhibition is expected, and body weight gain was actually inhibited by SCD-1 knockout in obesity model mice (Non-patent Literature 18).

Obesity and accumulation of excessive lipid cause life-style diseases such as arteriosclerosis and insulin resistance, so that SCD-1 inhibition is expected to improve life-style diseases. It is suggested that insulin resistance in SCD-1 knockout mice is improved by inhibition of glucose production and promotion of signal transduction downstream of an insulin receptor (Non-patent Literature 19), and it is reported that forced expression of SCD-1 decreased an ATP-binding Cassette Transporter A1 (ABCA1) protein in cells, and thus decreased efflux of cholesterol from cells (Non-patent Literature 20). These results suggest that SCD-1 inhibition has lipid modification effect and anti-obesity effect, and also has beneficial effects on arteriosclerosis and diabetes. Therefore, SCD-1 inhibitors are under development as new agents for life-style diseases.

### Citation List

### Patent Literature

Patent Literature 1: WO 2008/129951

### Non-patent Literature

Non-patent Literature 1: Biochemistry Vol. 77, 1412-1417
Non-patent Literature 2: Arterioscler Thromb Vasc Biol. 27, 2420-7
Non-patent Literature 3: Proc Natl Acad Sci USA. 102, 6086-91
Non-patent Literature 4: Arterioscler Thromb Vasc Biol. 15, 1688-94
Non-patent Literature 5: FASEB J. Epub ahead of print.
Non-patent Literature 6: Cell 2008 133: 1 (66-77)
Non-patent Literature 7: Hepatology 49, 306-17 (2009)
Non-patent Literature 8: J Biol Chem. 277, 19353-7 (2002)
Non-patent Literature 9: Genes Dev. 14, 2831-8 (2000)
Non-patent Literature 10: Metabolism. 56, 470-5 (2007)
Non-patent Literature 11: Journal of General Virology 89, 5, 1225-1230 (2008)
Non-patent Literature 12: Diabetes 54: 8, 2328-2335 (2005)
Non-patent Literature 13: Pharmacogenomics Journal 5: 5, 298-304 (2005)
Non-patent Literature 14: Curr Opin Lipidol. 19, 248-56 (2008)
Non-patent Literature 15: J Biol Chem. 275, 30132-8 (2000)
Non-patent Literature 16: J Lipid Res. 43, 1899-907 (2002)
Non-patent Literature 17: Proc Natl Acad Sci USA. 101, 6409-14 (2004)
Non-patent Literature 18: Diabetes. 56, 1228-39 (2008)
Non-patent Literature 19: J Clin Invest. 116, 1686-95 (2006)
Non-patent Literature 20: J Biol Chem. 278, 5813-20 (2003)

### Summary of the Invention

### Technical Problem

The present invention is intended to provide a pharmaceutical product for inhibiting expression of at least one lipid metabolism related mRNA selected from the group consisting of Angptl4 mRNA, SCD-1 mRNA, and SREBP1c mRNA. The present invention is also intended to provide a preventive and/or therapeutic agent for various diseases based on inhibition of expression of at least one lipid metabolism related mRNA selected from the group consisting of Angptl4 mRNA, SCD-1 mRNA, and SREBP1c mRNA.

### Solution to Problem

As a result of dedicated research for achieving the above-described purposes, the inventors have found that a compound represented by Formula (I) , its salt, or a solvate of any of them has a high inhibiting effect on expression of Angptl4 mRNA, SCD-1 mRNA, or SREBP1c mRNA, the substance has an effect of inhibiting production of Angpt14, SCD-1, or SREBP1c, and thus such a compound is effective for prevention and/or treatment of various diseases. The present invention has been completed on the basis of these findings.

More specifically, the present invention provides an agent for inhibiting expression of at least one lipid metabolism related mRNA selected from the group consisting of Angptl4 mRNA, SCD-1 mRNA, and SREBP1c mRNA, the agent comprising a compound represented by Formula (I), its salt, or a solvate of any of them as an active ingredient:

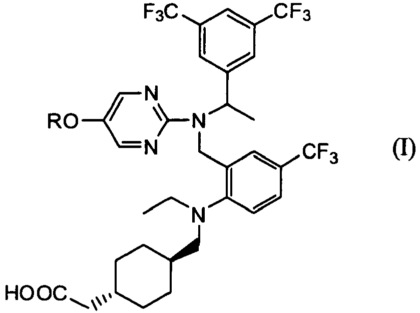

wherein R represents a lower alkylthio-lower alkyl group, a lower alkylsulfinyl-lower alkyl group, or a lower alkylsulfonyl-lower alkyl group.

The present invention also provides an agent for inhibiting production of at least one lipid metabolism related protein selected from the group consisting of Angptl4, SCD-1, and SREBP1c, the agent comprising a compound represented by Formula (I), its salt, or a solvate of any of them as an active ingredient.

The present invention also provides an agent for prevention, treatment or both of prevention and treatment of at least one disease selected from the group consisting of osteoporosis, fatty liver, non-alcoholic steatohepatitis, hepatitis C virus-associated adiposis, malignancy syndrome, extrapyramidal symptoms, diabetes, and obesity, the agent comprising a compound represented by Formula (I) , its salt, or a solvate of any of them as an active ingredient.

The present invention also provides a preventive and/or therapeutic agent for kidney disease having at least one symptom selected from the group consisting of lipid accumulation in the kidney, glomerulosclerosis, tubulointerstitial fibrosis, and proteinuria, the agent comprising a compound represented by Formula (I) , its salt, or a solvate of any of them as an active ingredient.

The present invention also provides an agent for inhibiting cancer metastasis or deterioration of myelin sheath function or myelin formation, the agent comprising a compound represented by Formula (I), its salt, or a solvate of any of them as an active ingredient.

The present invention also provides a compound represented by Formula (I), its salt, or a solvate of any of them for inhibiting expression of lipid metabolism related mRNA selected from the group consisting of Angptl4 mRNA, SCD-1 mRNA, and SREBP1c mRNA, or production of at least one lipid metabolism related protein selected from the group consisting of Angptl4, SCD-1, and SREBP1c.

The present invention also provides a compound represented by Formula (I), its salt, or a solvate of any of them for preventing and/or treating at least one disease selected from the group consisting of osteoporosis, fatty liver, non-alcoholic steatohepatitis, hepatitis C virus-associated adiposis, malignancy syndrome, extrapyramidal symptoms, diabetes, and obesity.

The present invention also provides a compound represented by Formula (I), its salt, or a solvate of any of them for preventing and/or treating kidney disease having at least one symptom selected from the group consisting of lipid accumulation in the kidney, glomerulosclerosis, tubulointerstitial fibrosis, and proteinuria.

The present invention also provides a compound represented by Formula (I), its salt, or a solvate of any of them for inhibiting cancer metastasis or deterioration of myelin sheath function or myelin formation.

The present invention also provides use of a compound represented by Formula (I), its salt, or a solvate of any of them for producing an agent for inhibiting expression of at least one lipid metabolism related mRNA selected from the group consisting of Angptl4 mRNA, SCD-1 mRNA, and SREBP1c mRNA, or production of at least one lipid metabolism related protein selected from the group consisting of Angptl4, SCD-1, and SREBP1c.

The present invention also provides use of a compound represented by Formula (I), its salt, or a solvate of any of them for producing a preventive and/or therapeutic agent for at least one disease selected from the group consisting of osteoporosis, fatty liver, non-alcoholic steatohepatitis, hepatitis C virus-associated adiposis, malignancy syndrome, extrapyramidal symptoms, diabetes, and obesity.

The present invention also provides use of a compound represented by Formula (I), its salt, or a solvate of any of them for producing a preventive and/or therapeutic agent for kidney disease having at least one symptom selected from the group consisting of lipid accumulation in the kidney, glomerulosclerosis, tubulointerstitial fibrosis, and proteinuria.

The present invention also provides use of a compound represented by Formula (I), its salt, or a solvate of any of them for producing an agent for inhibiting cancer metastasis or deterioration of myelin sheath function or myelin formation.

The present invention also provides a method for inhibiting expression of at least one lipid metabolism related mRNA selected from the group consisting of Angptl4 mRNA, SCD-1 mRNA, and SREBP1c mRNA, or production of at least one lipid metabolism related protein selected from the group consisting of Angptl4, SCD-1, and SREBP1c, the method including administration of an effective dose of a compound represented by Formula (I), its salt, or a solvate of any of them.

The present invention also provides a method for preventing and/or treating at least one disease selected from the group consisting of osteoporosis, fatty liver, non-alcoholic steatohepatitis, hepatitis C virus-associated adiposis, malignancy syndrome, extrapyramidal symptoms, diabetes, and obesity, the method including administration of an effective dose of a compound represented by Formula (I), its salt, or a solvate of any of them.

The present invention also provides a method for preventing and/or treating kidney disease having at least one symptom selected from the group consisting of lipid accumulation in the kidney, glomerulosclerosis, tubulointerstitial fibrosis, and proteinuria, the method including administration of an effective dose of a compound represented by Formula (I), its salt, or a solvate of any of them.

The present invention also provides a method for inhibiting cancer metastasis or deterioration of myelin sheath function or myelin formation, the method including administration of an effective dose of a compound represented by Formula (I), its salt, or a solvate of any of them.

### Effects of the Invention

As specifically disclosed in the below-described examples, a compound represented by Formula (I) has a strong inhibiting effect on expression of Angptl4 mRNA, SCD-1 mRNA, or SREBP1c mRNA. These compounds inhibit production of Angptl4, SCD-1, or SREBP1c, and thus are effective for prevention and/or treatment of various diseases caused by these proteins.

### Brief Description of Drawings

FIG. 1 shows the relative value of the expression level of Angptl4 mRNA after adding the compound according to the present invention (compound 2) and (4S,5R)-5-[3,5-bis(trifluoromethyl)phenyl]-3-({2-[4-fluoro-2-methoxy-5-(propane-2-yl)phenyl]-5-(trifluoromethyl)phenyl }methyl)-4-methyl-1,3-oxazolidine-2-one (Anacetrapib: therapeutic agent for dyslipidemia based on CETP inhibitory activity, see WO 2006/014357) at specified concentrations.
FIG. 2 shows the relative value of the expression level of SREBP1c mRNA after adding the compound according to the present invention (compound 2) and Anacetrapib.
FIG. 3 shows the relative value of the expression level of SCD-1 mRNA after adding the compound according to the present invention (compound 2) and Anacetrapib.

### Description of Embodiments

The active ingredient of the pharmaceutical product of the present invention is a compound represented by Formula (I), its salt, or a solvate of any of them. These compounds are described in Patent Literature 1, and are known to have inhibitory activity on cholesterol ester transfer protein (CETP) . However, their effects on the production of AngPt14, SCD-1, and SREBP1c are unknown.

In the present description, examples of the "lower alkyl" moiety in the "lower alkylthio-lower alkyl group", "lower alkylsulfinyl-lower alkyl group", and "lower alkylsulfonyl-lower alkyl group" include linear or branched alkyl having 1 to 6 carbon atoms (expressed as C₁-C₆ alkyl) . Examples of the "C₁-C₆ alkyl" include methyl, ethyl, n-propyl, isopropyl, n-butyl, isobutyl, sec-butyl, tert-butyl, n-pentyl, isopentyl, neopentyl, 2-methylbutyl, 1-ethylpropyl, n-hexyl, isohexyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 1-ethyl butyl, and 2-ethylbutyl.

In the present description, examples of the "lower alkylthio" moiety in the "lower alkylthio-lower alkyl group" include linear or branched alkylthio having 1 to 6 carbon atoms (expressed as C₁-C₆ alkylthio) . Examples of the "C₁-C₆ alkylthio" include methylthio, ethylthio, n-propylthio, isopropylthio, n-butylthio, isobutylthio, sec-butylthio, tert-butylthio, n-pentylthio, isopentylthio, neopentylthio, 2-methylbutyl thio, 1-ethylpropylthio, n-hexylthio, isohexylthio, 3-methylpentylthio, 2-methylpentylthio, 1-methylpentylthio, 3,3-dimethylbutylthio, 2,2-dimethylbutylthio, 1,1-dimethylbutylthio, 1,2-dimethylbutylthio, 1,3-dimethylbutylthio, 2,3-dimethylbutylthio, 1-ethylbutylthio, and 2-ethylbutylthio.

In the present description, examples of the "lower alkylsulfinyl" moiety in the "lower alkylsulfinyl-lower alkyl group" include linear or branched alkylsulfinyl having 1 to 6 carbon atoms (expressed as C₁-C₆ alkylsulfinyl). Examples of the "C₁-C₆ alkylsulfinyl" include methylsulfinyl, ethylsulfinyl, n-propylsulfinyl, isopropylsulfinyl, n-butylsulfinyl, isobutylsulfinyl, sec-butylsulfinyl, tert-butylsulfinyl, n-pentylsulfinyl, isopentylsulfinyl, neopentylsulfinyl, 2-methylbutylsulfinyl, 1-ethylpropylsulfinyl, n-hexylsulfinyl, isohexylsulfinyl, 3-methylpentylsulfinyl, 2-methylpentylsulfinyl, 1-methylpentylsulfinyl, 3,3-dimethylbutylsulfinyl, 2,2-dimethylbutylsulfinyl, 1,1-dimethylbutylsulfinyl, 1,2-dimethylbutylsulfinyl, 1,3-dimethylbutylsulfinyl, 2,3-dimethylbutylsulfinyl, 1-ethylbutylsulfinyl, and 2-ethylbutylsulfinyl.

In the present description, examples of the "lower alkylsulfonyl" moiety in the "lower alkylsulfonyl-lower alkyl group" include linear or branched alkylsulfonyl having 1 to 6 carbon atoms (expressed as C₁-C₆ alkylsulfonyl) . Examples of the "C₁-C₆ alkylsulfonyl" include methylsulfonyl, ethylsulfonyl, n-propylsulfonyl, isopropylsulfonyl, n-butylsulfonyl, isobutylsulfonyl, sec-butylsulfonyl, tert-butylsulfonyl, n-pentylsulfonyl, isopentylsulfonyl, neopentylsulfonyl, 2-methylbutylsulfonyl, 1-ethylpropylsulfonyl, n-hexylsulfonyl, isohexylsulfonyl, 3-methylpentylsulfonyl, 2-methylpentylsulfonyl, 1-methylpentylsulfonyl, 3,3-dimethylbutylsulfonyl, 2,2-dimethylbutylsulfonyl, 1,1-dimethylbutylsulfonyl, 1,2-dimethylbutylsulfonyl, 1,3-dimethylbutylsulfonyl, 2,3-dimethylbutylsulfonyl, 1-ethyl butylsulfonyl, and 2-ethylbutylsulfonyl.

The compound represented by Formula (I) may be any stereoisomer or any mixture of any stereoisomers, such as an optically pure isomer or any mixture of the isomer, a racemic, any geometric isomer, or any mixture of geometric isomers.

Examples of the stereoisomer of the compound represented by Formula (I) include the compounds represented by Formulae (II) and (III) :

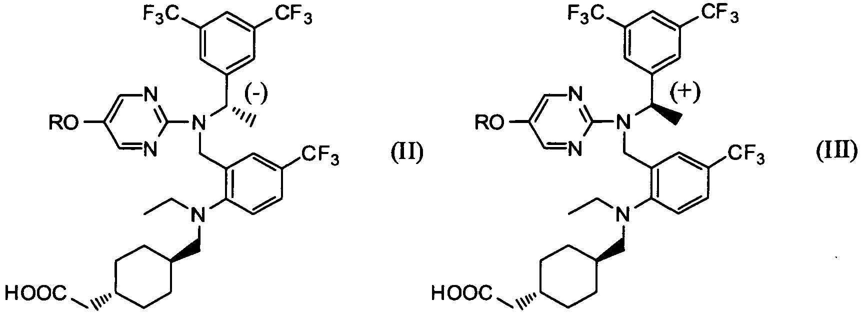

wherein R represents the same as above.

In Formulae (I) to (III), R is preferably a lower alkylsulfonyl-lower alkyl group, more preferably C₁ to C₆ alkylsulfonyl C₁ to C₆ alkyl groups, and particularly preferably a 2-(methylsulfonyl)ethyl group.

Among the compound represented by Formula (I) , so-called prodrugs, which are the compounds metabolized in the living body to be converted into the compound represented by Formula (I) , are also included. Examples of the group which can form the prodrugs of the compound represented by Formula (I) include the groups described in "Progress in Medicine, "LifescienceMedica, 1985, Vol. 5, p. 2157-2161, and the groups described in Bunshi Sekkei (Molecular Design) , p. 163-198, Vol. 7 of "Iyakuhin no Kaihatsu (Development of Pharmaceutical Products)," issued in 1990 by Hirokawa-Shoten Ltd.

Examples of preferred compounds in the present invention include
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid (compound 1),
(S)-(-)-trans-{4-[({2-[({l-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid (compound 2), and
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid (compound 3), but the scope of the invention will not be limited to these compounds.

Examples of the salt of the compound represented by Formula (I) include acid addition salts and base addition salts, and the salt is not particularly limited as long as it is pharmaceutically acceptable. Examples of the acid addition salt include acid addition salts with inorganic acids such as hydrochloride, hydrobromide, hydroiodide, sulfate, nitrate, and phosphate; acid addition salts with organic acids, such as benzoate, methanesulfonate, ethanesulfonate, benzenesulfonate, p-toluenesulfonate, maleate, fumarate, tartrate, citrate, and acetate. Examples of the base addition salt include base addition salts with metals such as sodium salt, potassium salt, lithium salt, calcium salt, and magnesium salt; amine salts such as ammonia, trimethylamine, triethylamine, pyridine, collidine, and lutidine; and base addition salts with organic bases such as ricin and arginine.

Examples of the solvent forming the solvate of the compound represented by Formula (I) or its salt include, but not limited to, water and physiologically acceptable organic solvents such as ethanol, hexane, and ethyl acetate. Examples of the active ingredient of the pharmaceutical product of the present invention include, but not limited to, hydrates.

The compound represented by Formula (I) may be produced by the method described in Patent Literature 1. The compound 1 preferred in the present invention is disclosed in Example 45 of Patent Literature 1. The compounds 2 and 3, which are enantiomers of the compound 1, may be produced, for example, from the compound 1 using a chiral column, or by subjecting a derivative of the compound 1 to preference crystallization method or the like to separate followed by derivation to the compound 2.

The pharmaceutical product comprising the compound represented by Formula (I) has a strong inhibitory effect on expression of Angptl4 mRNA, SCD-1 mRNA, or SREBP1c mRNA, and is effective for prevention and/or treatment of osteoporosis, fatty liver, non-alcoholic steatohepatitis, hepatitis C virus-associated adiposis, malignancy syndrome, extrapyramidal symptoms, diabetes, or obesity. In addition, the pharmaceutical product comprising the compound represented by Formula (I) is also effective for prevention and/or treatment of kidney disease having at least one symptom selected from the group consisting of lipid accumulation in the kidney, glomerulosclerosis, tubulointerstitial fibrosis, and proteinuria, or inhibition of cancer metastasis or deterioration of myelin sheath function or myelin formation.

Examples of the osteoporosis to which the compound represented by Formula (I) is applicable include primary osteoporosis and secondary osteoporosis. Examples of the primary osteoporosis include type I osteoporosis (postmenopausal osteoporosis), type II osteoporosis (regressive osteoporosis or senile osteoporosis), and idiopathic osteoporosis. Examples of inhibition of cancer metastasis to which the compound represented by Formula (I) is applicable include inhibition of cancer metastasis from mammary tissues to lung tissues.

Angptl, which is one of secreted glycoprotein having high homology to Angiopoietin playing an important role in angiogenesis, is a protein whose biological action is different from that of Angiopoietin. Angptl1 to Angptl6 have been identified, and it is reported that Angptl4 increased bone absorption by osteoclasts. The increase in bone absorption by osteoclasts causes osteoporosis, so that the inhibition of the expression of Angpt14 mRNA likely leads to the prevention and treatment of osteoporosis. On the other hand, it is reported that Angptl4 promoted metastasis of breast cancer to the lung, so that inhibition of expression of Angptl4 mRNA likely leads to inhibition of metastasis of certain cancer.

It is known that knockout of SREBP1c in ob/ob mice, which are obesity model mice, causes decrease of fatty acid synthase in the liver, and thus decrease of a triglyceride level in the liver. This fact suggests that activation of SREBP1c is correlated with fatty liver, and there is an actual report that administration of an agent increasing an expression level of SREBP1c enhanced fatty liver. Fatty liver can develop NASH, and SREBP1c is suggested to be a risk factor for NASH. It is reported that mice with forced expression of SREBP1c had accumulation of fat in the liver and hepatic fibrosis associated with aging, and showed a NASH-like condition. Accordingly, the compound represented by Formula (I) is likely effective for prevention and/or treatment of fatty liver or NASH.

The enhancement of the transcription of SREBP1c by hepatitis C virus non-structural protein 2 (HCVNS2) is considered to contribute to hepatitis C virus-associated adiposis, so that the compound represented by Formula (I) is likely effective for prevention and/or treatment of hepatitis C virus-associated adiposis. In addition, it has been proved that the increased expression of SREBP1c plays an important role in occurrence of lipid accumulation in the kidney, glomerulosclerosis, tubulointerstitial fibrosis, and proteinuria. Therefore, the compound represented by Formula (I) is likely effective for prevention and/or treatment of kidney disease having at least one symptom selected from the group consisting of lipid accumulation in the kidney, glomerulosclerosis, tubulointerstitial fibrosis, and proteinuria. Furthermore, deterioration of myelin sheath function or myelin formation, and malignancy syndrome or extrapyramidal symptoms, which are side effects of administration of psychotropic agents, are likely caused by the increased expression of SREBP1c, so that the compound represented by Formula (I) is likely effective for prevention and/or treatment of deterioration of myelin sheath function or myelin formation, and malignancy syndrome or extrapyramidal symptoms.

Examples of diabetes to which the compound according to the present invention is applicable include type 1 diabetes and type 2 diabetes. Examples of obesity to which the compound represented by Formula (I) is applicable include, visceral fat obesity and subcutaneous adipose obesity.

It is also reported that knockout of SCD-1 in the living body promoted β-oxidation of fatty acids through activation of AMP activated protein kinase (AMPK), and thus enhanced energy metabolism in the body, and there is an actual report that SCD-1 knockout in obesity model mice inhibited the body weight gain. Therefore, the compound represented by Formula (I) is likely effective for prevention and/or treatment of obesity. On the other hand, it is reported that the enhancement of signal transduction downstream of the insulin receptor improved insulin resistance in SCD-1 knockout mice, so that the compound represented by Formula (I) is likely effective for prevention and/or treatment of diabetes.

The pharmaceutical product of the present invention comprises the compound represented by Formula (I), its salt, or a solvate of any of them as an active ingredient. As the pharmaceutical product of the present invention, the active ingredient may be administered as it is, but preferably administered in the form of an oral or parenteral pharmaceutical composition which can be produced by a method well-known to those skilled in the art. Examples of the pharmaceutical composition suitable for oral administration include, but not limited to, tablets, capsules, powders, fine grains, granules, liquid, and syrups. Examples of the pharmaceutical composition suitable for parenteral administration include, but not limited to, injections such as intravenous injections and intramuscular injections, intravenous drips, suppositories, inhalants, eye drops, nasal drips, transdermal absorbents, and transmucosal absorbents.

The pharmaceutical composition may be produced with pharmacologically and pharmaceutically acceptable additives. Examples of the pharmacologically and pharmaceutically acceptable additive include, but not limited to, excipients, binders, fillers, disintegrating agents, surfactants, lubricants, dispersants, buffers, preservatives, flavoring agents, perfumes, film agents, and diluents.

The dose of the pharmaceutical product of the present invention is not particularly limited, and may be appropriately selected according to the disease type, the purpose of prevention or treatment, and the type of active ingredient, and may be appropriately increased or decreased according to various factors which should be normally taken into consideration, such as the body weight and age of the patient, symptoms, and administration route. For example, for oral administration cases, the dose of the compound represented by Formula (I) is from 0.1 mg to 1000 mg, preferably from 1 mg to 1000 mg, and more preferably from 1 mg to 500 mg a day for an adult. However, the dose may be appropriately selected by those skilled in the art, and will not be limited to the above-described range.

### Examples

The present invention is further described below with reference to examples, but the present invention will not be limited to these examples. The symbols used in the following examples represent the following meanings;
s: singlet
d: doublet
t: triplet
q: quartet
m: multiplet
br: broad
J: coupling constant
Hz: Hertz
CDCl₃: chloroform-d
¹H-NMR: proton nuclear magnetic resonance

### Preparation Example 1

The compound 1 was prepared in accordance with the method disclosed in Example 45 of WO 2008/129951. The compounds 2 and 3, which are a pair of enantiomers of the compound 1, were separated from the compound 1 using a chiral column under the following conditions.

Column: CHIRALCEL OD-H (4.6 x 250mm)
Flow rate: 1.0 mL/min
Detector: UV 242 nm
Temp.: 40°C
Mobile phase: Hexane/EtOH/TFA = 90/10/0.1
Retention time: (R)-(+)-form 21.3 min,(S)-(-)-form 23.7 min

### Compound 2

¹H-NMR (CDCl₃)δ: 0.80-0.96 (7H,m) , 1.38 (1H.m), 1.47 (3H, d, J=7.1Hz ),1.65-1.77(5H,m),2.19(2H,d,J=6.8Hz),2.72(1H,m),2.81-2.91(3 H,m),3.08(3H,s),3.45(2H,t,J=5.4Hz),4.44(2H,t,J=5.4Hz),4.62( 1H,d,J=17.1Hz),4.86(1H,d,J=17.1Hz),6.21(1H,q,J=7.1Hz),7.13( 1H,d,J=8.3Hz),7.19(1H,s),7.38(1H,d,J=8.3Hz),7.71(1H,s),7.73 (2H, s) , 8 . 15 (2H, s) .
[α] _{D}²⁰ =-46. 68 (c = 1 . 0, CHCl₃)

### Compound 3

¹H-NMR (CDCl₃) δ: same as the compound 2
[α]_{D}²⁰ = +48.92 (c = 1 . 0, CHCl₃)

Preparation Example 2: preparation of substantially optically pure (S) -enantiomer of the compound 2 by preferential crystallization
The outline of the method for preparing the substantially optically pure (S) -enantiomer of the compound 2 by preferential crystallization carried out by the inventors is described below as scheme 1. The absolute configurations of the respective compounds were determined from the absolute configuration of (R)-1-bromo-1-[3,5-bis(trifluoromethyl)phenyl]ethane, which had been confirmed in Step 1.
The optical purity of the (S) -enantiomer of the compound 2 ((S)-trans-{4-[({2-[({1-[3,5-bis(trifluoromethyl)phenyl] eth yl}{5-[2-(methylsulfonyl)ethoxy]pyrimidine-2-yl}amino)methy 1]-4-(trifluoromethyl)phenyl}(ethyl)amino)methyl]cyclohexyl }acetic acid) obtained in Step 6 was determined by chiral HPLC analysis under the conditions described in Preparation Example 1.
Further, the optical purity of 1-bromo-1-[3,5-bis(trifluoromethyl)phenyl] ethane obtained in the step 1 and trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}benz yl acetate ester obtained in steps 4 and 5 was individually determined by chiral HPLC analysis under the following conditions.

Chiral HPLC analysis conditions for
1-bromo-1-[3,5-bis(trifluoromethyl)phenyl]ethane
Column: CHIRALPAK AS-RH
Mobile phase: ethanol /water = 60/40
Flow rate: 0.5 mL/min
Column temperature: 25°C
Detection wavelength: 220 nm
Retention time: first peak/ 21.8 min ((R) enantiomer), second peak/ 26.0 min ((S)-enantiomer)

Chiral HPLC analysis conditions for
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}benz yl acetate ester
Column: CHIRALCEL OD-H
Mobile phase: hexane/ethanol = 80/20
Flow rate: 1.0 mL/min
Column temperature: 40°C
Detection wavelength: 242 nm
Retention time: first peak/11.3 min ((R)-enantiomer), second peak/ 13.0 min ((S)-enantiomer)

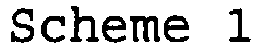

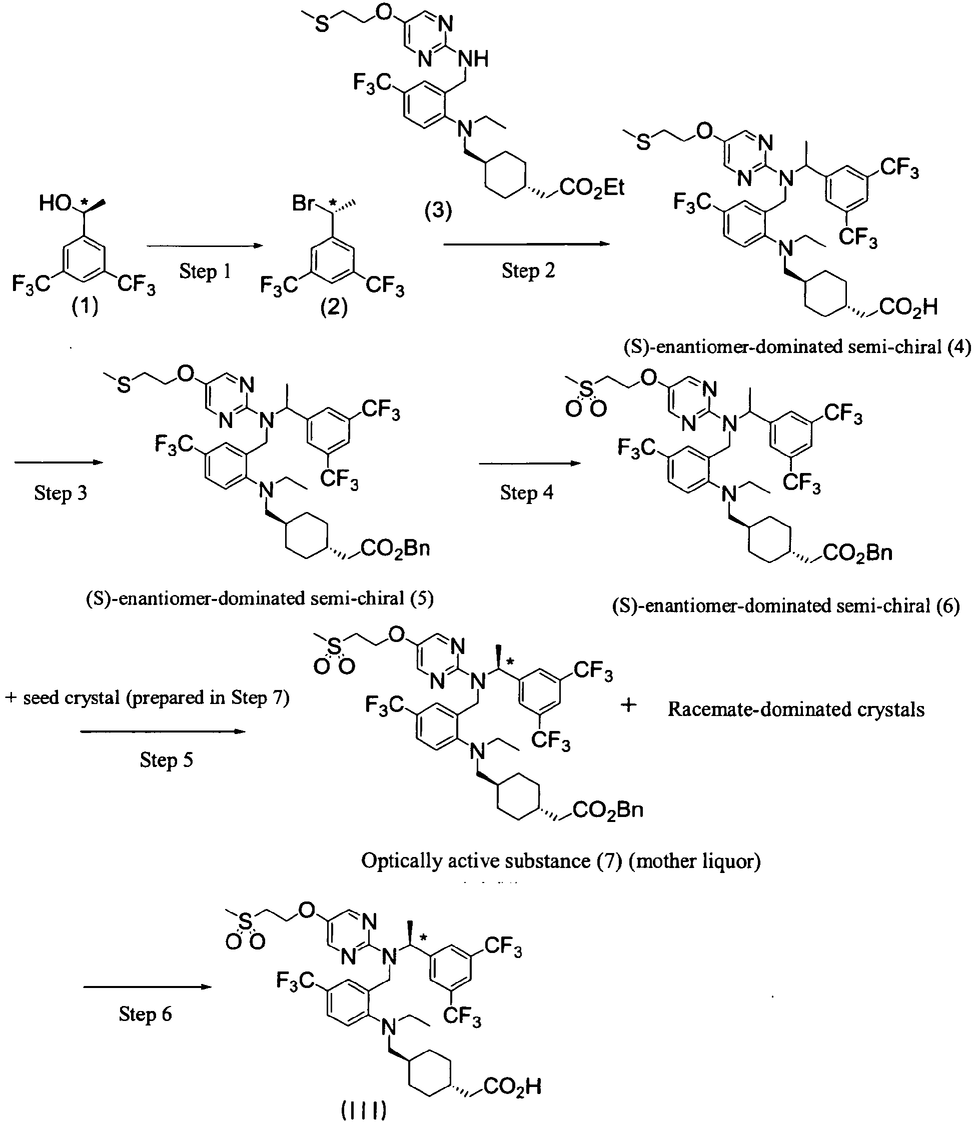

(In the scheme, Et represents an ethyl group, and Bn represents a benzyl group.)

### Step 1: preparation of

### (R)-1-bromo-1-[3,5-bis(trifluoromethyl)phenyl]ethane

(R)-1-bromo-1-[3,5-bis(trifluoromethyl)phenyl]ethane was prepared by the following method 1-(a), and the absolute configuration was confirmed as described below. More specifically, the
(R)-1-bromo-1-[3,5-bis(trifluoromethyl)phenyl]ethane thus obtained was converted into
(S)-1-[3,5-bis(trifluoromethyl)phenyl]ethylamine, and the symbol of the actual specific optical rotation was compared with that of the commercially available reference standard of (S)-1-[3,5-bis (trifluoromethyl)phenyl] ethylamine, whose absolute configuration had been known.
In addition,
(R)-1-bromo-1-[3,5-bis(trifluoromethyl)phenyl]ethane was also prepared by the method following 1-(b).

### 1-(a): preparation of (R)-1-bromo-1-[3,5-bis(trifluoromethyl)phenyl]ethane No. 1

In argon atmosphere, 1,2-dibromo-1,1,2,2-tetrachloroethane (7.57 g, 23.2 mmol) was dissolved in toluene (12.5 mL) , triphenylphosphine (6.1 g, 23.2 mmol) was added at 0°C, and stirred for 30 minutes. To the solution, a toluene solution (12.5 mL) of
(S)-1-[3,5-bis(trifluoromethyl)phenyl]ethanol (1) (5.0 g, 19.4 mmol, > 99.5% ee) was added dropwise over a period of 10 minutes or more at 0°C, the mixture was heated to room temperature, and stirred for 1 hour at the temperature. To the reaction liquid was added n-hexane (25 mL), and filtered through Celite. The filtrate was sequentially washed with water, saturated sodium bicarbonate water, and saturated saline solution, dried with sodium sulfate, and then evaporated under reduced pressure. The residue thus obtained was distillated under reduced pressure (56°C, 0.7 mmHg), thereby obtaining 5.52 g of (R)-1-bromo-1-[3,5-bis(trifluoromethyl)phenyl]ethane (2) in the form of a colorless oil (yield: 88.6%).

Chiral HPLC analysis: optical purity > 99.5% ee (main peak: first peak), degree of conversion ≥ 99%
[α]_{D}²⁵ +59.1 (c = 1.03, CHCl₃) ¹H-NMR (CDCl₃) δ: 2.08 (3H, d, J = 7.1 Hz), 5.21 (1H, q, J = 7.1 Hz), 7.81 (1H, s), 7.87 (2H, s)

Confirmation of absolute configuration of (R)-1-bromo-1-[3,5-bis(trifluoromethyl)phenyl]ethane Sodium azide (64.4 mg, 0.990 mmol) was added to the N,N-dimethylformamide solution (1 mL) of the (R)-1-bromo-1-[3,5-bis(trifluoromethyl)phenyl]ethane (2) (106 mg, 0.336 mmol, 99% ee) which had been obtained in the above-described 1- (a) , and stirred for 4 hours at -18 to -15°C. The reaction solution was extracted by ethyl acetate/n-hexane (1:1) and water, the organic layer was washed with saturated saline solution, dried with anhydrous sodium sulfate, and then concentrated under reduced pressure, thereby obtaining 111.5 mg of 1-[3,5-bis(trifluoromethyl)phenyl]ethyl azide (crude product: 111.5 mg).

¹H-NMR (CDCl₃) δ: 1.61 (3H, d, J = 6.8 Hz), 4.79 (1H, q, J = 6.8 Hz), 7.78 (2H, s), 7.84 (1H, s)

The 1-[3,5-bis(trifluoromethyl)phenyl]ethyl azide (crude product: 111.5 mg) thus obtained was dissolved in methanol (6 mL), mixed with palladium-fibroin (18 mg), and substituted to hydrogen gas replacement, and stirred for 1 hour at room temperature. The solution was filtered through Celite, the filtrate was concentrated under reduced pressure, and the residue thus obtained was purified by silica gel column chromatography (chloroform : methanol = 50:1 to 5:1), thereby obtaining 77.6 mg of
1-[3,5-bis (trifluoromethyl)phenyl] ethylamine in the form of a colorless oil (yield: 91%, two steps).

¹H-NMR (CDCl₃) δ: 1.42 (3H, d, J = 6.8 Hz), 1.58 (2H, br-s), 4.30 (1H, q, J = 6.8 Hz), 7.75 (1H, s), 7.85 (2H, s)

The specific optical rotation of the 1-[3,5-bis(trifluoromethyl)phenyl]ethylamine thus obtained was as described below.
[α] _{D}²⁵ -15.9 (c = 1.31, CHCl₃)

The specific optical rotation of the commercially available reference standard of
((S)-1-[3,5-bis(trifluoromethyl)phenyl]ethylamine (Central Glass Co., Ltd.: Lot. 0102000: optical purity: 99% ee)) was as follows:
[α]_{D}²⁵ -15.9 (c = 1.15, CHCl₃)

The symbol of the actual specific optical rotation agreed with the symbol of the commercially available reference standard, indicating that the 1-[3,5-bis(trifluoromethyl)phenyl]ethylamine thus obtained is an (S)-enantiomer. This amine was prepared from 1-bromo-1-[3,5-bis (trifluoromethyl) phenyl] ethane through the nucleophilic substitution reaction of azide ion, indicating that the 1-bromo-1-[3,5-bis(trifluoromethyl)phenyl]ethane obtained in the above-described 1-(a) is an (R)-enantiomer.

### 1-(b): preparation of (R)-1-bromo-1-[3,5-bis(trifluoromethyl)phenyl]ethane No. 2

In argon atmosphere, phosphorus tribromide (157.3 g, 0.58 mol) was added dropwise to (S)-1-[3,5-bis(trifluoromethyl)phenyl]ethanol (1) (300 g, 1.16 mol, 96% ee) in a water bath at 20°C or lower, and stirred for 30 minutes at 19 to 22°C. The reaction liquid was cooled, hydrogen bromide (30% acetic acid solution) (228 mL, 1.16 mol) was added dropwise at 0°C or lower, and stirred for 16 hours at 13 to 15°C. The reaction liquid was added to ice water, and extracted with n-hexane (3 L x 2). The organic layers were combined, subsequently washed with saturated sodium bicarbonate water (3 L) and saturated saline solution (3 L), dried with anhydrous magnesium sulfate, and then concentrated under reduced pressure (90 to 100 mm Hg), thereby obtaining 389.2 g of crude product. The crude product thus obtained was purified by column chromatography (silica gel 900 g, developing solvent: n-hexane), thereby obtaining 349.8 g of (R)-1-bromo-1-[3,5-bis(trifluoromethyl)phenyl]ethane (2) in the form of a colorless oil (yield: 93.8%).
As described below, in the chiral HPLC analysis, the first peak appeared as the main peak, indicating that the 1-bromo-1-[3,5-bis(trifluoromethyl)phenyl] ethane prepared in 1-(b) is also an (R)-enantiomer as that prepared in 1-(a).

Chiral HPLC analysis: optical purity > 93.9% ee (main peak: first peak), degree of conversion: 97.8% ¹H - NMR (CDCl₃) δ :
2.08(3H,d,J=7.1Hz),5.21(1H,q,J=7.1Hz),7.81(1H,s),7.87(2H,s)

### Step 2: preparation of (S)-enantiomer-dominated semi-chiral of trans-{4-[({2-[({1-[3,5-bis(trifluoromethyl)phenyl]ethyl}{5 -[2-(methylthio)ethoxy]pyrimidine-2-yl}amino)methyl]-4-(tri fluoromethyl)phenyl}(ethyl)amino)methyl]cyclohexyl}acetic acid

In argon atmosphere, NaH (60% in oil, 119 g, 2.98 mol) was added to the anhydrous tetrahydrofuran (THF, 2.26 L) solution of
trans-[4-([(ethyl){2-[({5-[2-(methylthio)ethoxy]pyrimidine-2-yl}amino)methyl]-4-(trifluoromethyl)phenyl}amino]methyl)c yclohexyl] ethyl acetate (3) (565.4 g, 0.99 mol), which had been synthesized by the method described in Patent Literature 2 (WO 2008/129951), under cooling with ice, and stirred for 1 hour at room temperature. The reaction liquid was cooled to -30°C, and
anhydrous N,N-dimethylformamide (4.53 L) solution of the (R)-1-bromo-1-[3,5-bis(trifluoromethyl)phenyl]ethane (2) obtained in the process 1 (682 g, 1.99 mol, 93.9% ee) was added dropwise to the reaction liquid with the internal temperature of the reaction system kept at -15°C or lower, and stirred for 5 hours at -15°C to -1°C. The reaction liquid was added to the mixed solution of ice water (35 L) and toluene (30 L), citric acid was added until the pH reached 6.9, and the organic layer was separated.

The aqueous layer was extracted with two portions of toluene (20 L), the organic layers were combined, dried with anhydrous magnesium sulfate, and concentrated under reduced pressure, thereby obtaining a crude product. The crude product was dissolved in ethanol (8 L), 2M NaOH aqueous solution (1.24 L, 2.48 mol) was added under cooling with ice, and stirred for 3.5 hours at 50°C. 1M HCl aqueous solution was added to the reaction liquid under cooling with ice until the pH reached 5.4, the mixture was added to water (25 L), and extracted two portions of ethyl acetate (22 L). The organic layer was washed with saturated saline solution (12 L), dried with anhydrous magnesium sulfate, concentrated under reduced pressure, and the residue thus obtained was purified by column chromatography (silica gel 21 g, developing solvent: heptane/acetone = 7/1 → 3/1), thereby obtaining semi-chiral (4) of trans-{4-[({2-[({1-[3,5-bis(trifluoromethyl)phenyl]ethyl}{5 -[2-(methylthio)ethoxy]pyrimidine-2-yl}amino)methyl]-4-(tri fluoromethyl)phenyl}(ethyl)amino)methyl]cyclohexyl}acetic acid (yellow oil, 744.1 g, yield: 96%).

The (R)-1-bromo-1-[3,5-bis (trifluoromethyl)phenyl] ethane (2), whose absolute configuration had been confirmed as described in the above-described Step 1, was used as the raw material, and nucleophilic substitution reaction by the amine (3) proceeded, indicating that the semi-chiral (4) thus obtained is dominated by (S)-enantiomer.

¹H - NMR (CDCl₃) δ:
0. 85 - 0. 96 (7H,m), 1.35 - 1.45 (4H,m) , 1.60 - 1. 78 (5H,m) , 2.18 - 2.2 1(5H,m),2.69(1H,m),2.81-2.91(5H,m),4.16(2H,q,J=6.8Hz),4.61 (1H,d,J=17.1Hz),4.85(1H,d,J=17.1Hz),6.22(1H,q,J=6.8Hz),7.11 (1H,d,J=8.6Hz),7.23(1H,s),7.37(1H,d,J=8.3Hz),7.70(1H,s),7.7 3(2H,s),8.14(2H,s)

### Step 3: preparation of (S)-enantiomer-dominated semi-chiral of trans-{4-[({2-[({1-[3,5-bis(trifluoromethyl)phenyl]ethyl}{5 -[2-(methylthio)ethoxy]pyrimidine-2-yl}amino)methyl]-4-(tri fluoromethyl)phenyl}(ethyl)amino)methyl]cyclohexyl}benzyl acetate ester

In argon atmosphere, benzyl alcohol (113.1 g, 1.05 mol) andWSC·HCl (200.5 g, 1.05 mol) and DMAP (11.9 g, 98 mmol) were added to the anhydrous dichloroethane (11.6 L) solution of the (S)-enantiomer-dominated semi-chiral (4) (744.1 g, 0.95 mol) of trans-{4-[({2-[({1-[3,5-bis(trifluoromethyl)phenyl]ethyl}{5 -[2-(methylthio)ethoxy]pyrimidine-2-yl}amino)methyl]-4-(tri fluoromethyl)phenyl}(ethyl)amino)methyl]cyclohexyl}acetic acid, which had been obtained in Step 2, under cooling with ice, and stirred at room temperature overnight. Water (10 L) was added to the reaction liquid, extracted with chloroform (19 L, 14 L), the organic layer was washed with saturated saline solution (12 L), dried with anhydrous magnesium sulfate, concentrated under reduced pressure, and the residue thus obtained was purified by column chromatography (silica gel 28 g, developing solvent: heptane/ethyl acetate = 6/1), thereby obtaining the semi-chiral (5) of trans-{4-[({2-[({1-[3,5-bis(trifluoromethyl)phenyl]ethyl}{5 -[2-(methylthio)ethoxy]pyrimidine-2-yl}amino)methyl]-4-(tri fluoromethyl)phenyl}(ethyl)amino)methyl]cyclohexyl}benzyl acetate ester (yellow oil, 745.8 g, yield: 90%).

The semi-chiral (5) thus obtained is dominated by (S)-enantiomer as the semi-chiral (4).

¹H - NMR (CDCl₃) δ: 0.87 - 0.95 (7H,m) , 1.37(1H.m) 1.43 (3H, d, J=7. 1Hz) 1.65 - 1.77 (5 H,m),2.20(2H,d,J=6.8Hz),2.22(3H,s),2.66-2.71(2H,m),2.82-2 .91(4H,m),4.15(2H,t,J=6.6Hz),4.62(1H,d,J=17.1Hz),4.85(1H,d, J=17.1Hz),5.10(2H,s),6.21(1H,q,J=7.1Hz), 7.10(1H,d,J=8.3Hz), 7.22(1H,s),7.28-7.38(6H,m),7.70(1H,s),7.73(2H,s),8.14(2H,s )

### Step 4: preparation of (S)-enantiomer-dominated semi-chiral of trans-{4-[({2-[({1-[3,5-bis(trifluoromethyl)phenyl]ethyl} {5 -[2-(methylsulfonyl)ethoxy]pyrimidine-2-yl}amino)methyl]-4-(trifluoromethyl)phenyl}(ethyl)amino)methyl]cyclohexyl}benz yl acetate ester

In argon atmosphere, tantalum pentachloride (31. 3 g, 87.3 mmol) and 30% hydrogen peroxide solution (496 mL, 4.38 mol) were added to the 2-propanol (15.2 L) solution of the semi-chiral (5) (745.8 g, 0.87 mol) of the (S)-enantiomer-dominated trans-{4-[({2-[({1-[3,5-bis (trifluoromethyl)phenyl] ethyl} {5 -[2-(methylthio)ethoxy]pyrimidine-2-yl}amino)methyl]-4-(tri fluoromethyl)phenyl}(ethyl)amino)methyl]cyclohexyl}benzyl acetate ester, which had been obtained in Step 3, at room temperature, and stirred for 5 hours. The reaction liquid was quenched with saturated sodium hydrogensulfite aqueous solution (3.1 L), water (15 L) was added, extracted with chloroform (14 L, 12 L), the organic layer was washed with saturated saline solution (20 L), dried with anhydrous magnesium sulfate, concentrated under reduced pressure, and the residue thus obtained was purified by column chromatography (silica gel 26 kg, developing solvent: heptane/ethyl acetate = 3/1 → 2/1), thereby obtaining semi-chiral (6) of trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}benz yl acetate ester (yellow amorphous, 619.5 g, yield: 79%).

The semi-chiral (6) thus obtained is dominated by (S)-enantiomer as the semi-chiral (4) and semi-chiral (5).

### Chiral HPLC analysis: optical purity: 67.7% ee (main peak: second peak)

¹H - NMR (CDCl₃ ) δ :
0.87 - 0.96 (7H,m) , 1.38 (1H,m) , 1.45 (3H, d, J=7. 1Hz) , 1. 65 - 1.80 (5 H,m),2.21(2H,d,J=6.6Hz),2.69(1H,m),2.81-2.91(3H,m),3.08(3H ,s),3.44(2H,t,J=5.4Hz),4.44(2H,t,J=5.4Hz),4.64(1H,d,J=17.1H z),4.86(1H,d,J=17.3Hz),5.10(2H,s),6.19(1H,q,J=6.9Hz),7.12(1 H,d,J=8.3Hz),7.19(1H,s),7.30-7.39(6H,m),7.71(lH,s),7.72(2H ,s),8.16(2H,s)

### Step 5: preparation of substantially optically pure (S)-trans-{4-[({2-[({1-[3,5-bis(trifluoromethyl)phenyl]ethy 1}{5-[2-(methylsulfonyl)ethoxy]pyrimidine-2-yl}amino)methyl ]-4-(trifluoromethyl)phenyl}(ethyl)amino)methyl]cyclohexyl} benzyl acetate ester

The semi-chiral (6) (111.7 g, 123.7 mmol, 67.7% ee) of the (S)-enantiomer-dominated trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}benz yl acetate ester, which had been obtained in Step 4, was dissolved in ethanol (825 mL) , and 2.0 mg of the seed crystal, which had been prepared in advance (racemic crystals prepared in the below-described Step 7) was added at 15°C to 20°C, and stirred for 21 hours at the temperature, and 3 hours at 0°C. The precipitate was removed by filtration, washed with cold ethanol (165 mL), and then the mother liquor was concentrated under reduced pressure, thereby obtaining substantially optically pure trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}benz yl acetate ester (7) (yellow amorphous, 66.38 g, yield: 59%).
The trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}benz yl acetate ester (7) thus obtained is (S) -enantiomer, because it had been prepared by removing the racemate-dominated crystals by filtration from the (S)-enantiomer-dominated semi-chiral (6).

### Chiral HPLC analysis: optical purity > 99 % ee (main peak: second peak)

[α]_{D}²⁰ -42.36 (c = 1.0 w/v%, CHCl₃)

The optical purity of the racemate-dominated crystals removed by filtration was 22% ee as measured by chiral HPLC analysis (43.39 g, yield: 39%).

### Step 6: preparation of substantially optically pure (S)-trans-{4-[({2-[({1-[3,5-bis (trifluoromethyl)phenyl]ethy 1}{5-[2-(methylsulfonyl)ethoxy]pyrimidine-2-yl}amino)methyl 1-4-(trifluoromethyl)phenyl}(ethyl)amino)methyl]cyclohexyl} acetic acid

In a nitrogen atmosphere, 10% Pd-C (wet) (3.4 g) was added to an ethanol (340 mL) solution of the (S)-trans-{4-[({2-[({1-[3,5-bis(trifluoromethyl)phenyl]ethy l}{5-[2-(methylsulfonyl)ethoxy]pyrimidine-2-yl}amino)methyl ]-4-(trifluoromethyl)phenyl}(ethyl)amino)methyl]cyclohexyl} benzyl acetate ester (7) (34.2 g, 37.88 mmol, > 99% ee), which had been obtained in Step 5, the mixture was substituted by hydrogen gas replacement, and then stirred for 2 hours at room temperature. The reaction suspension was filtered through Celite, washed with ethanol (50 mL), and the wash liquid was concentrated under reduced pressure, thereby obtaining substantially optically pure trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid (compound 2) (white amorphous, 31.78 g, yield: 100%).
The compound thus obtained is a levo-rotatory compound as indicated by the specific optical rotation shown below. The compound thus obtained is also an (S)-enantiomer, because it was prepared by deprotecting the ester groups of the (S) -benzyl ester (7) .

### Chiral HPLC analysis: optical purity > 99% ee (main peak: the second peak)

[α] _{D}²⁰ - 46.68 (c = 1.0, CHCl₃)
IR(ATR)cm⁻¹:2921,1706,1479,1279,1134
¹H - NMR (CDCl₃) δ:0.80 - 0.96 (7H,m) , 1.38 (1H.m),1.47(3H,d,J=7.1H z),1.65-1.77(5H,m),2.19(2H,d,J=6.8Hz),2.72(1H,m),2.81-2.9 1(3H,m),3.08(3H,s),3.45(2H,t,J=5.2Hz),4.44(2H,q,J=5.4Hz),4. 62(1H,d,J=17.1Hz),4.86(1H,d,J=17.4Hz),6.21(1H,q,J=7.1Hz),7. 13(1H,d,J=8.3Hz),7.19(1H,s),7.38(1H,d,J=6.6Hz),7.71(1H,s),7 .73(2H,s),8.15(2H,s)

### Step 7: preparation of racemic seed crystals of trans-{4-[({2-[({1-[3,5-bis(trifluoromethyl)phenyl]ethyl}{5 -[2-(methylsulfonyl)ethoxy]pyrimidine-2-yl}amino)methyl] -4-(trifluoromethyl)phenyl}(ethyl)amino)methyl]cyclohexyl}benz yl acetate ester

Benzyl alcohol (2.93 g, 27.07 mmol), DMAP (300 mg, 2.46 mmol) and WSC-HCl (5.19 g, 27. 07 mmol) were added to an anhydrous dichloromethane (200 mL) solution of
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid (racemic compound (1)) (20 g, 24.61 mmol), which had been synthesized by the method described in Example 45 of Patent Literature 2 (WO 2008/129951) under cooling with ice, the mixture was heated to room temperature, and stirred for 16 hours. Water (100 mL) was added to the reaction liquid, extracted with chloroform (500 mL), an organic layer was washed with 2M aqueous hydrochloric acid solution (100 mL) and saturated saline solution (100 mL), dried with anhydrous magnesium sulfate, and then concentrated under reduced pressure. The residue thus obtained was purified by column chromatography (silica gel 350 g, developing solvent: n-hexane/ethyl acetate = 3/1 → 1/1), thereby obtaining
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}benz yl acetate ester (21.15 g, yield: 95.2%) in the form of a white amorphous.

The trans-{4-[({2-[({1-[3,5-bis(trifluoromethyl)phenyl]ethyl}{5 -[2-(methylthio)ethoxy]pyrimidine-2-yl}amino)methyl]-4-(tri fluoromethyl)phenyl}(ethyl)amino)methyl]cyclohexyl}benzyl acetate ester (7.9 g) was dissolved in ethanol (40 mL), and stirred for 15 hours at room temperature. The precipitate thus obtained was collected by filtration, washed with cold ethanol (20 mL), and dried for 4 hours at 60°C under reduced pressure, thereby obtaining racemic crystals of trans-{4-[({2-[({1-[3,5-bis(trifluoromethyl)phenyl]ethyl}{5 -[2-(methylsulfonyl)ethoxy]pyrimidine-2-yl}amino)methyl] -4-(trifluoromethyl)phenyl}(ethyl)amino)methyl]cyclohexyl}benz yl acetate ester (white crystalline powder, 6.98 g, yield: 88.4%).

In Preparation Example 2, the compound having moderate optical purity (about 50 to 90% ee, preferably about 70 to 90% ee) , which is obtained as a result of the decrease of the optical purity caused by partial racemization, is referred to as a "semi-chiral." In the semi-chiral, when the compound with S asymmetric carbon is present in more amount than the compound with R asymmetric carbon, the semi-chiral is referred to as "(S)-enantiomer-dominated semi-chiral."

### Test Example 1

The test compound 2 was added to the HepG2 cells of human liver cancer cell line, cultured for 24 hours, and then the expression level of Angptl4 mRNA was measured by real-time quantitative RT-PCR. More specifically, the HepG2 cells were seeded on a 24-well plate at a concentration of 2 x 10⁵ cells/well, and cultured overnight. The test compound 2 was dissolved in dimethyl sulfoxide (DMSO) at concentrations of 0.1 mM, 1 mM, and 10 mM, and added to the culture solution at the rate of 1 to 1000. The mixture was cultured for 8 hours in a CO₂ incubator at 37°C, and then 500 µL of ISOGEN (Nippon Gene Co., Ltd., catalogue No. 31-02501) was added, and the total RNA was extracted. From the extracted total RNA, cDNA was synthesized using High Capacity cDNA Reverse Transcription kit (Applied Biosystems, catalogue No. 4368813). The expression level of human Angptl4 mRNA was measured using a primer 5'-CTCAGATGGAGGCTGGACAGT-3' (SEQ ID NO. 1) and an antisense primer 5'-TGATGCTATGCACCTTCTCCA-3' (SEQ ID NO. 2), which are specific primers to human Angptl4, and Fast SYBR Green master mix (Applied Biosystems, catalogue No. 4385614). The measurement instrument was 7900HT Fast Realtime PCR system.

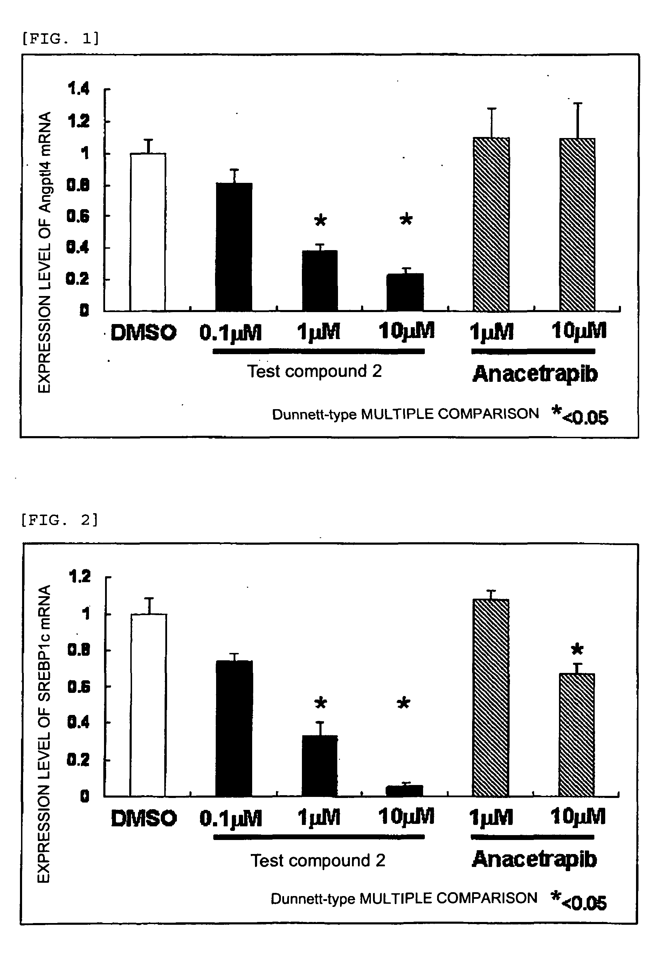

The measurement value was corrected by the expression level of β-Actin mRNA. The expression level of Angptl4 mRNA in the cells mixed with DMSO alone was set at 1, and the expression level of Angptl4 mRNA in the cells mixed with the test compound 2 was calculated as relative value. The results are shown in FIG. 1. As shown in FIG. 1, this effect was not caused by the other CETP inhibition drug Anacetrapib, indicating that this effect is likely not due to the CETP inhibitory effect, but is characteristic to the compound of the present invention.

The results of the above pharmacological test proved that the compound expressed by Formula (I) has a strong and long-lasting inhibitory effect on expression of Angptl4 mRNA.

### Test Example 2

The test compound 2 was added to the HepG2 cells of human liver cancer cell line, cultured for 24 hours, and then the expression level of SREBP1c mRNA was measured by real-time quantitative RT-PCR. More specifically, the HepG2 cells were seeded on a 24-well plate at a concentration of 2 x 10⁵ cells/well, and cultured overnight. The test compound 2 was dissolved in dimethyl sulfoxide (DMSO) at concentrations of 0.1 mM, 1 mM, and 10 mM, and added to the culture solution at the rate of 1 to 1000 . The mixture was cultured for 8 hours in a CO₂ incubator at 37°C, and then 500 µL of ISOGEN (Nippon Gene Co., Ltd., catalogue No. 31-02501) was added, and the total RNA was extracted. From the extracted total RNA, cDNA was synthesized using High Capacity cDNA Reverse Transcription kit (Applied Biosystems, catalogue No. 4368813). The expression level of human SREBPlc mRNA was measured using a TaqMan probe 5'-TCGCGGAGCCATGGATTGCACT-3' (SEQ ID NO. 3), a sense primer: 5' -GGTAGGGCCAACGGCCT-3' (SEQ ID NO. 4) , and an antisense primer 5'-CTGTCTTGGTTGTTGATAAGCTGAA-3' (SEQ ID NO. 5), which are specific to human SREBP1c, and TaqMan Fast Universal PCR Master Mix (Applied Biosystems, catalogue No. 4367846). The measurement instrument was 7900HT Fast Realtime PCR system.

The measurement value was corrected by the expression level of β-Action mRNA. The expression level of SREBP1c mRNA in the cells mixed with DMSO alone was set at 1, and the expression level of SREBP1c mRNA in the cells mixed with the test compound 2 was calculated as relative value. The results are shown in FIG. 2. As shown in FIG. 2, the influence of test compound 2 on decrease of the expression level of SREBP1c mRNA was evidently stronger than that of Anacetrapib, but the CETP inhibitory effects of test compound 2 and Anacetrapib were equal, so that the effect of decreasing the expression level of SREBP1c mRNA is likely independent of the CETP inhibitory effect.

The above results of the pharmacological test indicate that the compound expressed by Formula (I) has a strong and long-lasting inhibitory effect on expression of SREBP1c mRNA.

### Test Example 3

The test compound 2 was added to the HepG2 cells of human liver cancer cell line, cultured for 24 hours, and then the expression level of SCD-1 mRNA was measured by real-time quantitative RT-PCR. More specifically, the HepG2 cells were seeded on a 24-well plate at a concentration of 2 x10⁵ cells/well, and cultured overnight. The test compound 2 was dissolved in dimethyl sulfoxide (DMSO) at concentrations of 0.1 mM, 1 mM, and 10 mM, and added to the culture solution at the rate of 1 to 1000. The mixture was cultured for 8 hours in a CO₂ incubator at 37°C, and then 500 µL of ISOGEN (Nippon Gene Co., Ltd., catalogue No. 31-02501) was added, and the total RNA was extracted. From the extracted total RNA, cDNA was synthesized using High Capacity cDNA Reverse Transcription kit (Applied Biosystems, catalogue No. 4368813). The expression level of human SCD-1 was measured using a primer 5' -TGTTCGTTGCCACTTTCTTG-3' (SEQ ID NO. 6) , an antisense primer 5'-AGCTCCAAGTGAAACCAGGA-3' (SEQ ID NO. 7), which are specific to human SCD-1, and Fast SYBR Green master mix (Applied Biosystems, catalogue No. 4385614). The measurement instrument was 7900HT Fast Realtime PCR system.

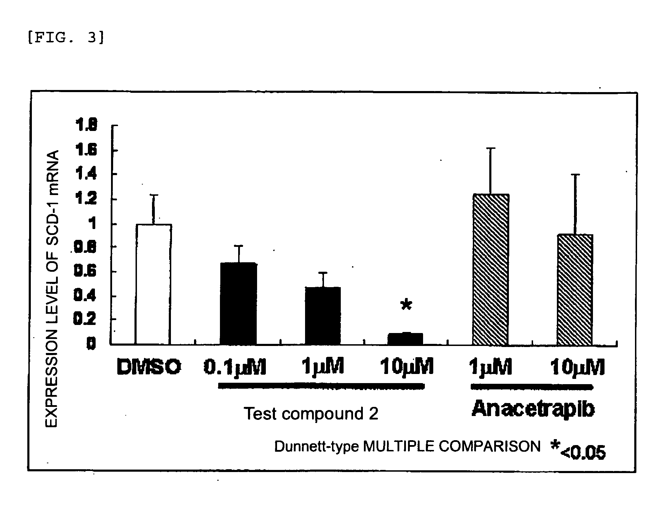

The measurement value was corrected by the expression level of β-Actin mRNA. The expression level of SCD-1 mRNA in the cells mixed with DMSO alone was set at 1, and the expression level of SCD-1 mRNA in the cells mixed with the test compound 2 was calculated as relative value. The results are shown in FIG. 3. As shown in FIG. 3, the influence of test compound 2 on decrease of the expression level of SCD-1 mRNA was evidently stronger than that of Anacetrapib, but the CETP inhibitory effects of the test compound 2 and Anacetrapib were equal, so that the effect of decreasing the expression level of SCD-1 mRNA is likely independent of the CETP inhibitory effect.

The above results of the pharmacological test indicate that the compound represented by Formula (I) has a strong and long-lasting inhibitory effect on expression of SCD-1 mRNA.

### Industrial Applicability

The compound represented by Formula (I) or a pharmaceutical product comprising the same has a strong inhibitory effect on expression of Angptl4 mRNA, SCD-1 mRNA, or SREBP1c mRNA. These compounds inhibit production of Angptl4, SCD-1, or SREBP1c, and thus are, as described above, effective for prevention and/or treatment of various diseases caused by any of these proteins.

## Claims

1. An agent for inhibiting expression of at least one lipid metabolism related mRNA selected from the group consisting of Angptl4 mRNA, SCD-1 mRNA, and SREBP1c mRNA, the agent comprising a compound represented by Formula (I), its salt, or a solvate of any of them as an active ingredient: 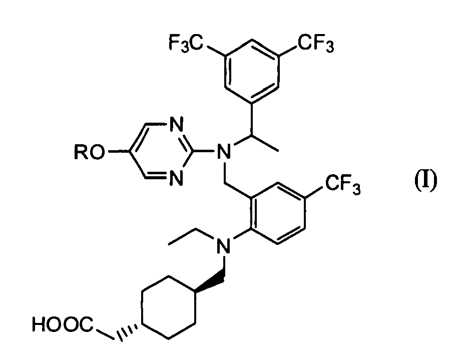 wherein R represents a lower alkylthio-lower alkyl group, a lower alkylsulfinyl-lower alkyl group, or a lower alkylsulfonyl-lower alkyl group.

2. The agent of claim 1, wherein the compound represented by Formula (I) is trans-{4-[({2-[({1-[3,5-bis(trifluoromethyl)phenyl]ethyl} {5 -[2-(methylsulfonyl)ethoxy]pyrimidine-2-yl}amino)methyl] -4-(trifluoromethyl)phenyl}(ethyl)amino)methyl]cyclohexyl}acet ic acid, (S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid, or (R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.

3. An agent for inhibiting production of at least one lipid metabolism related protein selected from the group consisting of Angptl4, SCD-1, and SREBP1c, the agent comprising a compound expressed by Formula (I), its salt, or a solvate of any of them as an active ingredient: 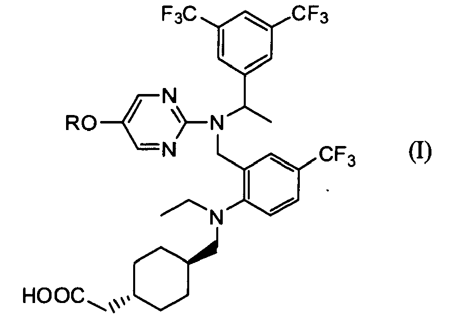 wherein R represents a lower alkylthio-lower alkyl group, a lower alkylsulfinyl-lower alkyl group, or a lower alkylsulfonyl-lower alkyl group.

4. The agent of claim 3, wherein the compound represented by Formula (I) is
trans-{4-[({2-[({1-[3,5-bis(trifluoromethyl)phenyl]ethyl}{5 - [2- (methylsulfonyl) ethoxy]pyrimidine-2-yl}amino)methyl] -4-(trifluoromethyl)phenyl}(ethyl)amino)methyl]cyclohexyl}acet ic acid,
(S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid, or
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.

5. An agent for inhibiting cancer metastasis in a living body, the agent comprising a compound represented by Formula (I), its salt, or a solvate of any of them as an active ingredient: 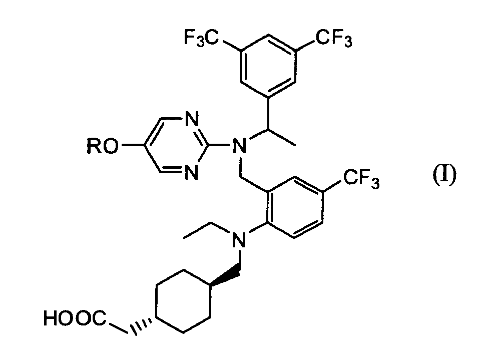 wherein R represents a lower alkylthio-lower alkyl group, a lower alkylsulfinyl-lower alkyl group, or a lower alkylsulfonyl-lower alkyl group.

6. The agent of claim 5, wherein inhibition of cancer metastasis is inhibition of cancer metastasis from mammary tissues to lung tissues.

7. The agent of claim 5 or 6, wherein the compound represented by Formula (I) is
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid, or
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl} (ethyl)amino)methyl]cyclohe xyl}acetic acid.

8. A preventive and/or therapeutic agent for at least one disease selected from the group consisting of osteoporosis, fatty liver, non-alcoholic steatohepatitis, hepatitis C virus-associated adiposis, malignancy syndrome, extrapyramidal symptoms, diabetes, and obesity, the agent comprising a compound represented by Formula (I), its salt, or a solvate of any of them as an active ingredient: 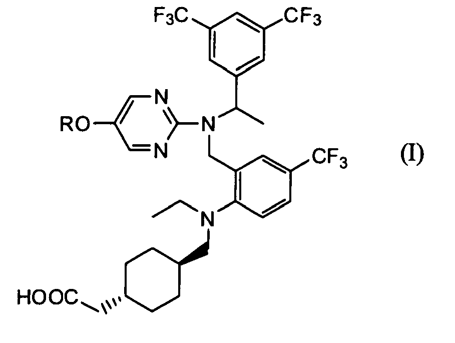 wherein R represents a lower alkylthio-lower alkyl group, a lower alkylsulfinyl-lower alkyl group, or a lower alkylsulfonyl-lower alkyl group.

9. The preventive and/or therapeutic agent of claim 8, wherein the compound is
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid, or
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl} (ethyl)amino)methyl]cyclohe xyl}acetic acid.

10. A preventive and/or therapeutic agent for kidney disease having at least one symptom selected from the group consisting of lipid accumulation in the kidney, glomerulosclerosis, tubulointerstitial fibrosis, and proteinuria, the agent comprising a compound represented by Formula (I), its salt, or a solvate of any of them as an active ingredient: 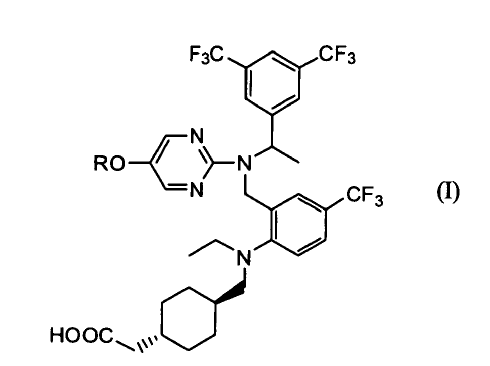 wherein R represents a lower alkylthio-lower alkyl group, a lower alkylsulfinyl-lower alkyl group, or a lower alkylsulfonyl-lower alkyl group.

11. The agent of claim 10, wherein the compound is
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid, or
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.

12. An agent for inhibiting deterioration of myelin sheath function or myelin formation, the agent comprising a compound represented by Formula (I), its salt, or a solvate of any of them as an active ingredient: 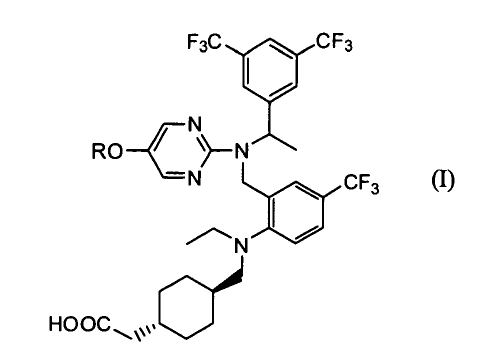 wherein R represents a lower alkylthio-lower alkyl group, a lower alkylsulfinyl-lower alkyl group, or a lower alkylsulfonyl-lower alkyl group.

13. The agent of claim 12, wherein the compound is
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid, or
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.

14. A compound represented by Formula (I), its salt, or a solvate of any of them for inhibiting expression of at least one lipid metabolism related mRNA selected from the group consisting of Angptl4 mRNA, SCD-1 mRNA, and SREBP1c mRNA: 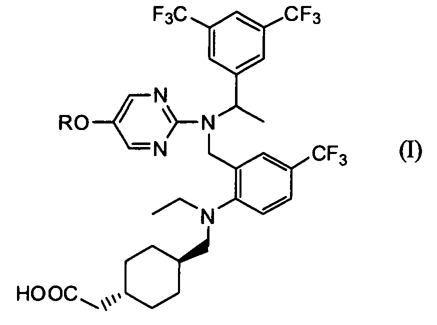 wherein R represents a lower alkylthio-lower alkyl group, a lower alkylsulfinyl-lower alkyl group, or a lower alkylsulfonyl-lower alkyl group.

15. The compound, its salt, or a solvate of any of them of claim 14, wherein the compound is
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid, or
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.

16. A compound represented by Formula (I), its salt, or a solvate of any of them for inhibiting production of at least one lipid metabolism related protein selected from the group consisting of Angptl4, SCD-1, and SREBP1c: 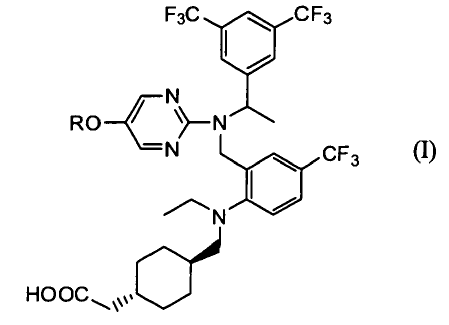 wherein R represents a lower alkylthio-lower alkyl group, a lower alkylsulfinyl-lower alkyl group, or a lower alkylsulfonyl-lower alkyl group.

17. The compound, its salt, or a solvate of any of them of claim 16, wherein the compound is
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid, or
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.

18. A compound represented by Formula (I), its salt, or a solvate of any of them for inhibiting cancer metastasis in the living body: 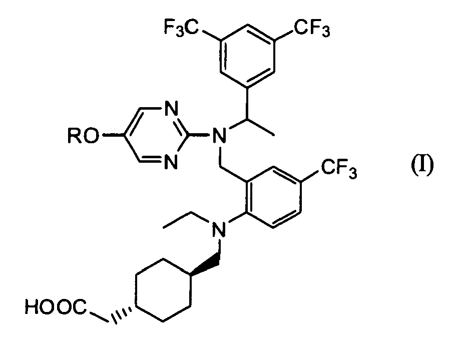 wherein R represents a lower alkylthio-lower alkyl group, a lower alkylsulfinyl-lower alkyl group, or a lower alkylsulfonyl-lower alkyl group.

19. The compound, its salt, or a solvate of any of them of claim 18, wherein inhibition of cancer metastasis is inhibition of cancer metastasis from mammary tissues to lung tissues.

20. The compound, its salt, or a solvate of any of them of claim 18 or 19, wherein the compound is trans-{4-[({2-[({1-[3,5-bis(trifluoromethyl)phenyl]ethyl}{5
-[2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid, or
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.

21. A compound represented by Formula (I), its salt, or a solvate of any of them for preventing and/or treating at least one disease selected from the group consisting of osteoporosis, fatty liver, non-alcoholic steatohepatitis, hepatitis C virus-associated adiposis, malignancy syndrome, extrapyramidal symptoms, diabetes, and obesity: 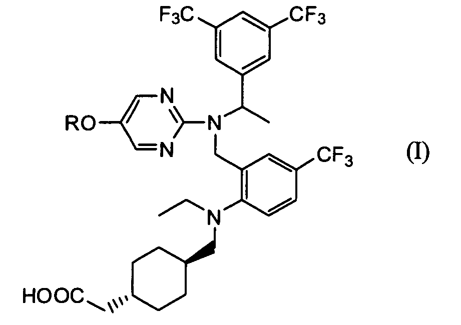 wherein R represents a lower alkylthio-lower alkyl group, a lower alkylsulfinyl-lower alkyl group, or a lower alkylsulfonyl-lower alkyl group.

22. The compound, its salt, or a solvate of any of them of claim 21, wherein the compound is
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid, or
(R)-(+)-trans-{4-[({2-[(({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.

23. A compound represented by Formula (I), its salt, or a solvate of any of them for preventing and/or treating kidney disease having at least one symptom selected from the group consisting of lipid accumulation in the kidney, glomerulosclerosis, tubulointerstitial fibrosis, and proteinuria: 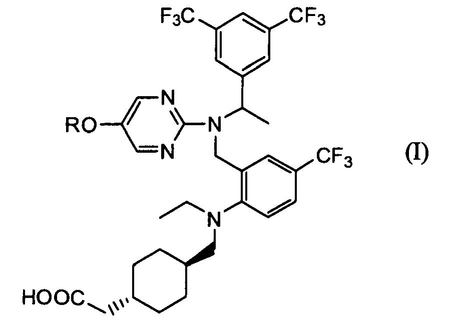 wherein R represents a lower alkylthio-lower alkyl group, a lower alkylsulfinyl-lower alkyl group, or a lower alkylsulfonyl-lower alkyl group.

24. The compound, its salt, or a solvate of any of them of claim 23, wherein the compound is
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2- [({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid, or
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.

25. The compound represented by Formula (I), its salt, or a solvate of any of them for inhibiting deterioration of myelin sheath function or myelin formation: 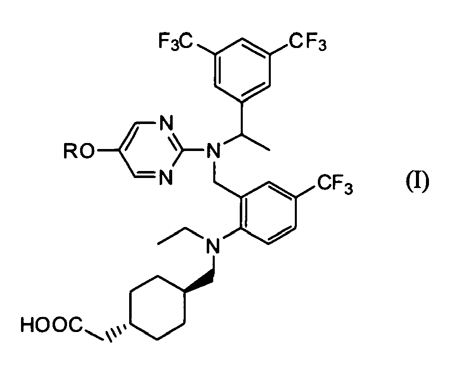 wherein R represents a lower alkylthio-lower alkyl group, a lower alkylsulfinyl-lower alkyl group, or a lower alkylsulfonyl-lower alkyl group.

26. The compound, its salt, or a solvate of any of them of claim 25, wherein the compound is
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid, or
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.

27. Use of a compound represented by Formula (I), its salt, or a solvate of any of them for producing an agent for inhibiting expression of at least one lipid metabolism related mRNA selected from the group consisting of Angptl4 mRNA, SCD-1 mRNA, and SREBP1c mRNA: 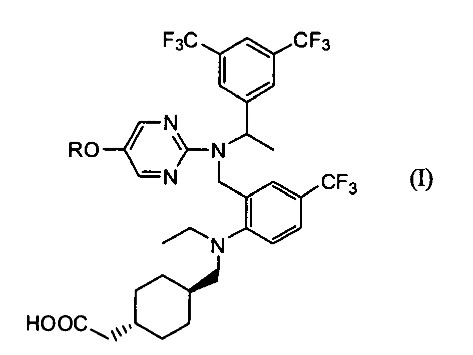 wherein R represents a lower alkylthio-lower alkyl group, a lower alkylsulfinyl-lower alkyl group, or a lower alkylsulfonyl-lower alkyl group.

28. The use of claim 27, wherein the compound is
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl} (ethyl)amino)methyl]cyclohe xyl}acetic acid, or
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.

29. Use of a compound represented by Formula (I), its salt, or a solvate of any of them for producing an agent for inhibiting production of at least one lipid metabolism related protein selected from the group consisting of Angptl4, SCD-1, and SREBP1c: 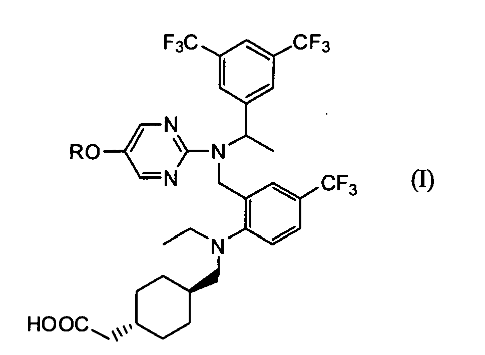 wherein R represents a lower alkylthio-lower alkyl group, a lower alkylsulfinyl-lower alkyl group, or a lower alkylsulfonyl-lower alkyl group.

30. The use of claim 29, wherein the compound is
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid, or
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.

31. Use of a compound represented by Formula (I), its salt, or a solvate of any of them for producing an agent for inhibiting cancer metastasis in the living body: 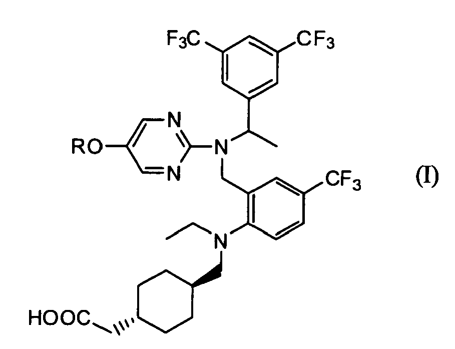 wherein R represents a lower alkylthio-lower alkyl group, a lower alkylsulfinyl-lower alkyl group, or a lower alkylsulfonyl-lower alkyl group.

32. The use of claim 31, wherein the inhibition of cancer metastasis is inhibition of cancer metastasis from mammary tissues to lung tissues.

33. The use of claim 31 or 32, wherein the compound is
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid, or
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.

34. Use of a compound represented by Formula (I), its salt, or a solvate of any of them for producing a preventive and/or therapeutic agent for at least one disease selected from the group consisting of osteoporosis, fatty liver, non-alcoholic steatohepatitis, hepatitis C virus-associated adiposis, malignancy syndrome, extrapyramidal symptoms, diabetes, and obesity: 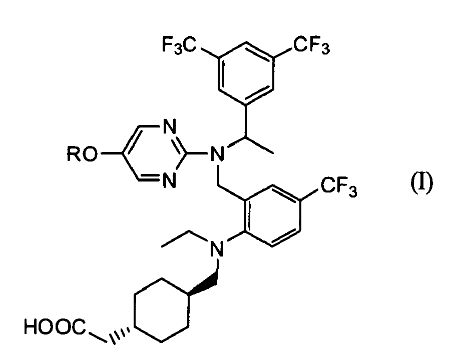 wherein R represents a lower alkylthio-lower alkyl group, a lower alkylsulfinyl-lower alkyl group, or a lower alkylsulfonyl-lower alkyl group.

35. The use of claim 34, wherein the compound is
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid, or
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.

36. Use of a compound represented by Formula (I), its salt, or a solvate of any of them for producing a preventive and/or therapeutic agent for kidney disease having at least one symptom selected from the group consisting of lipid accumulation in the kidney, glomerulosclerosis, tubulointerstitial fibrosis, and proteinuria: 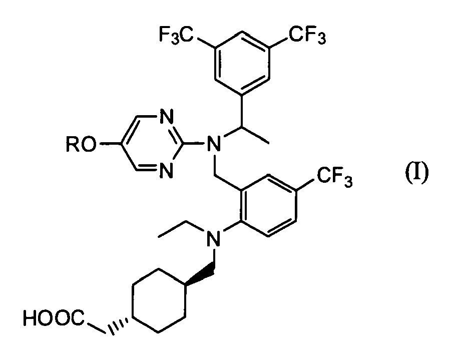 wherein R represents a lower alkylthio-lower alkyl group, a lower alkylsulfinyl-lower alkyl group, or a lower alkylsulfonyl-lower alkyl group.

37. The use of claim 36, wherein the compound is
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid, or
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.

38. Use of a compound represented by the Formula (I), its salt, or a solvate of any of them for producing an agent for inhibiting deterioration of myelin sheath function or myelin formation: 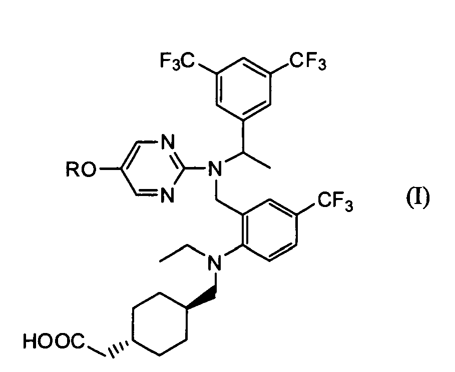 wherein R represents a lower alkylthio-lower alkyl group, a lower alkylsulfinyl-lower alkyl group, or a lower alkylsulfonyl-lower alkyl group.

39. The use of claim 38, wherein the compound is
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl} (ethyl)amino)methyl]cyclohe xyl}acetic acid, or
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.

40. A method for inhibiting expression of at least one lipid metabolism related mRNA selected from the group consisting of Angptl4 mRNA, SCD-1 mRNA, and SREBP1c mRNA, the method comprising administering an effective dose of the compound represented by Formula (I), its salt, or a solvate of any of them: 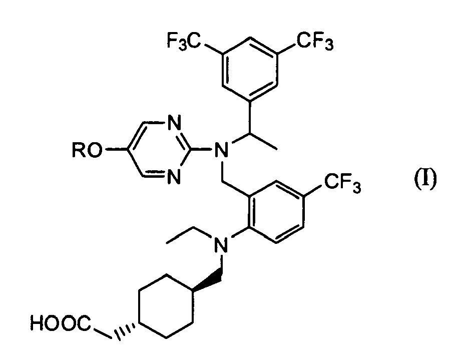 wherein R represents a lower alkylthio-lower alkyl group, a lower alkylsulfinyl-lower alkyl group, or a lower alkylsulfonyl-lower alkyl group.

41. The method of claim 40, wherein the compound is
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl} (ethyl)amino)methyl]cyclohe xyl}acetic acid, or
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.

42. A method for inhibiting production of at least one lipid metabolism related protein selected from the group consisting of Angptl4, SCD-1, and SREBP1c, the method comprising administering an effective dose of a compound expressed by Formula (I), its salt, or a solvate of any of them: 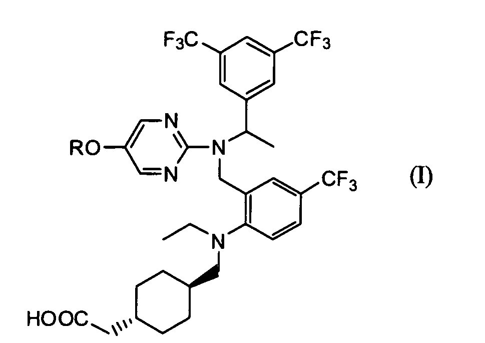 wherein R represents a lower alkylthio-lower alkyl group, a lower alkylsulfinyl-lower alkyl group, or a lower alkylsulfonyl-lower alkyl group.

43. The method of claim 42, wherein the compound is
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid, or
(R)-(+)-trans-{4-[({2-({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.

44. A method for inhibiting cancer metastasis in the living body, the method comprising administering an effective dose of a compound represented by Formula (I), its salt, or a solvate of any of them: 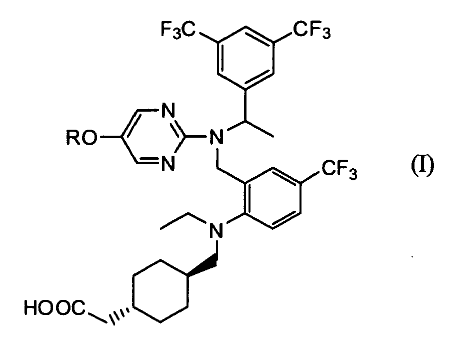 wherein R represents a lower alkylthio-lower alkyl group, a lower alkylsulfinyl-lower alkyl group, or a lower alkylsulfonyl-lower alkyl group.

45. The method of claim 44, wherein the inhibition of cancer metastasis is inhibition of cancer metastasis from mammary tissues to lung tissues.

46. The method of claim 44 or 45, wherein the compound represented by Formula (I) is
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid, or
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.

47. A method for preventing and/or treating at least one disease selected from the group consisting of osteoporosis, fatty liver, non-alcoholic steatohepatitis, hepatitis C virus-associated adiposis, malignancy syndrome, extrapyramidal symptoms, diabetes, and obesity, the method comprising administering an effective dose of a compound represented by Formula (I), its salt, or a solvate of any of them: 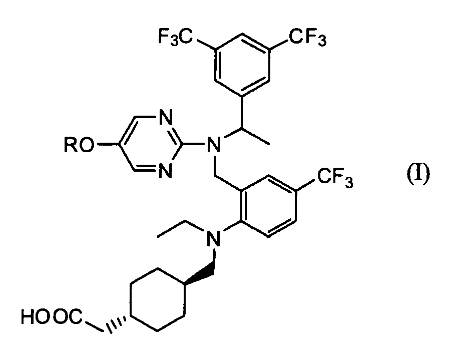 wherein R represents a lower alkylthio-lower alkyl group, a lower alkylsulfinyl-lower alkyl group, or a lower alkylsulfonyl-lower alkyl group.

48. The method of claim 47, wherein the compound is
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl} (ethyl)amino)methyl]cyclohe xyl}acetic acid, or
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.

49. A method for preventing and/or treating kidney disease having at least one symptom selected from the group consisting of lipid accumulation in the kidney, glomerulosclerosis, tubulointerstitial fibrosis, and proteinuria, the method comprising administering an effective dose of a compound expressed by Formula (I), its salt, or a solvate of any of them: 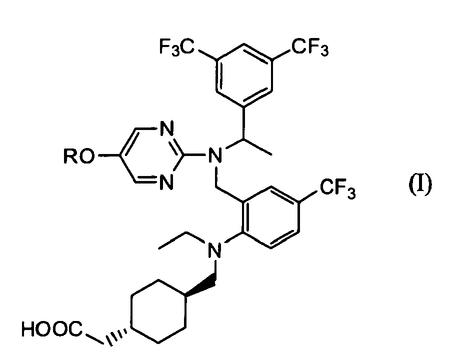 wherein R represents a lower alkylthio-lower alkyl group, a lower alkylsulfinyl-lower alkyl group, or a lower alkylsulfonyl-lower alkyl group.

50. The method of claim 49, wherein the compound is
trans-{4-[({2-[({1-[3,5-bis(trifluoromethyl)phenyl)ethyl}{5 - [2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2-[({1-[3,5-bis (trifluoromethyl)phenyl] ethyl}{5-[2-(methylsulfonyl)ethoxy]pyrimidine-2-yl}amino)me thyl]-4-(trifluoromethyl)phenyl} (ethyl)amino)methyl]cyclohe xyl}acetic acid, or
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.

51. A method for inhibiting deterioration of myelin sheath function or myelin formation, the method comprising administering an effective dose of a compound represented by Formula (I), its salt, or a solvate of any of them: 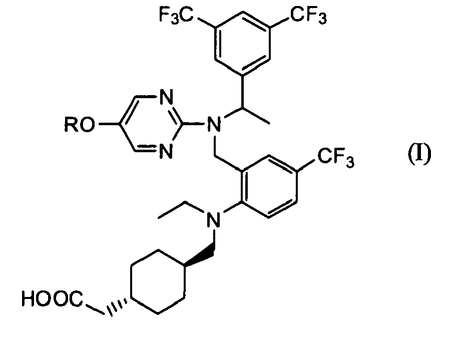 wherein R represents a lower alkylthio-lower alkyl group, a lower alkylsulfinyl-lower alkyl group, or a lower alkylsulfonyl-lower alkyl group.

52. The method of claim 51, wherein the compound is
trans-{4-[({2-[({1-[3,5-bis(trifluoromethyl)phenyl]ethyl}{5 -[2-(methylsulfonyl)ethoxy]pyrimidine-2-yl}amino)methyl]-4-(trifluoromethyl)phenyl}(ethyl)amino)methyl]cyclohexyl}acet ic acid,
(S)-(-)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid, or
(R)-(+)-trans-{4-[({2-[({1-[3,5-bis(trifluoromethyl)phenyl] ethyl}{5-[2-(methylsulfonyl)ethoxy]pyrimidine-2-yl}amino)me thyl]-4-(trifluoromethyl)phenyl}(ethyl)amino)methyl]cyclohe xyl}acetic acid.


SEQUENCE LISTING

<110> Kowa Co., Ltd.

<120> Medicine For Suppressing Expression Of Lipids
metabolism-associated mRNA

<130> KW0247

<150> JP2010-224490
<151> 2010-10-04
<150> JP2010-224563
<151> 2010-10-04
<150> JP2010-224564
<151> 2010-10-04

<160> 7

<170> PatentIn version 3.1

<210> 1
<211> 21
<212> DNA
<213> Artificial sequence

<220>
<223> oligonucleotide based on Angptl4 gene

<400> 1
ctcagatgga ggctggacag t
21

<210> 2
<211> 21
<212> DNA
<213> Artificial sequence

<220>
<223> oligonucleotide based on Angptl4 gene

<400> 2
tgatgctatg caccttctcc a
21

<210> 3
<211> 22
<212> DNA
<213> Artificial sequence

<220>
<223> oligonucleotide based on SREBP1c gene

<400> 3

tcgcggagcc atggattgca ct

22

<210> 4

<211> 17

<212> DNA

<213> Artificial sequence

<220>

<223> oligonucleotide based on SREBP1c gene

<400> 4

ggtagggcca acggcct

17

<210> 5

<211> 25

<212> DNA

<213> Artificial sequence

<220>

<223> oligonucleotide based on SREBP1c gene

<400> 5

ctgtcttggt tgttgataag ctgaa

25

<210> 6
<211> 20
<212> DNA
<213> Artificial sequence

<220>
<223> oligonucleotide based on SCD-1 gene

<400> 6
tgttcgttgc cactttcttg
20

<210> 7
<211> 20
<212> DNA
<213> Artificial sequence

<220>
<223> oligonucleotide based on SCD-1 gene

<400> 7
agctccaagt gaaaccagga
20